# EUROPEAN PATENT SPECIFICATION

(11) **EP 3 466 951 B1**
(45) Date of publication and mention of the grant of the patent: **19.01.2022**
(21) Application number: 18205889.1
(22) Date of filing: 23.07.2015
(51) Int. Cl.: C07D 471/14, C07D 461/00

(54) **A PROCESS FOR THE PREPARATION OF CGMP-PHOSPHODIESTERASE INHIBITOR AND ORAL PHARMACEUTICAL FORMULATION COMPRISING TADALAFIL CO-PRECIPITATES**
VERFAHREN ZUR HERSTELLUNG DES CGMP-PHOSPHODIESTERASEHEMMERS UND ORALE PHARMAZEUTISCHE FORMULIERUNG MIT TADALAFIL-CO-PRÄZIPITATEN
PROCÉDÉ DE PRÉPARATION D'INHIBITEUR DE CGMP-PHOSPHODIESTÉRASE ET FORMULATION PHARMACEUTIQUE ORALE COMPRENANT DES CO-PRÉCIPITÉS DE TADALAFIL

(30) Priority: 23.07.2014 SI 201400267
(43) Date of publication of application: 10.04.2019
(62) Divisional of application: 15739630.0
(73) Proprietor: KRKA, d.d., Novo mesto, 8000 Novo mesto (SI)
(72) Inventor: Baric, Matej, 8000 Novo mesto (SI); Benkic, Primoz, 8000 Novo mesto (SI); Bombek, Sergeja, 8000 Novo mesto (SI); Krasovec, Dusan, 8000 Novo mesto (SI); Skrabanja, Vida, 8000 Novo mesto (SI); Vrecer, Franc, 8000 Novo mesto (SI); Bukovec, Polona, 8000 Novo mesto (SI); Hudovornik, Grega, 8000 Novo mesto (SI); Kroselj, Vesna, 8000 Novo mesto (SI)
(74) Representative: Hoffmann Eitle

(56) References cited:
- EP-A1- 0 828 479
- US-A1- 2012 189 694

## Description

### FIELD OF THE INVENTION

The present invention relates to a method for production co-precipitate of CGMP-phosphodiesterase inhibitor tadalafil and to solid oral pharmaceutical formulations comprising tadalafil co-precipitate.

### BACKGROUND OF THE INVENTION

Tadalafil, chemically known as (6R-trans)-6-(1,3-benzodioxol-5-il)-2,3,6,7,12,12a-hexahydro-2-methyl-pyrazino[1', 2':1,6]pyrido[3,4-b]indole-1,4-dione, is a potent and selective inhibitor of the cyclic guanosine monophosphate (cGMP) - specific phosphodiesterase enzyme PDE5. It is shown below as structural formula I:

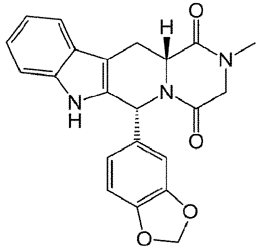

Tadalafil is marketed under the tradename CIALIS^{®} and is used for the treatment of erectile dysfunction. The product is available as a film-coated tablet for oral administration containing 2.5, 5, 10 and 20 mg of active ingredient and the following inactive ingredients: lactose monohydrate, hydroxypropylcellulose, sodium lauryl sulfate, croscarmellose sodium, microcrystalline cellulose, magnesium stearate, hypromellose, triacetin, titanium dioxide (E171), iron oxide (E172) and talc.

Tadalafil is practically insoluble in water and very slightly soluble in organic solvent such as ethanol, methanol and acetone.

Problems associated with low solubility of tadalafil in ethanol and most of other organic solvents resulted in the need of large quantities of solvents required to perform synthesis and crystallization of tadalafil at industrial scale, which have unwanted technological, environmental and economical impact.

US Patent No. 5 859 006 describes the synthesis of the tadalafil and its intermediate (A) which involves reacting D-tryptophan methyl ester with a piperonal in the presence of dichloromethane and trifluoroacetic acid which provides a mixture of desired cis and undesired trans isomer of intermediate A with poor selectivity. The isomers are further separated by column chromatography. The cis isomer is further reacted with chloroacetyl chloride in chloroform, providing another intermediate of tadalafil (B) which reacted with methylamine to give tadalafil of formula (1) in methanol slurry requiring an additional purification step by flash chromatography.

An improved process in the synthesis of tadalafil via modified Pictet-Spengler reaction is described in WO 04/011463 in which D-tryptophan methyl ester hydrochloride and piperonal are condensed in anhydrous isopropyl alcohol to provide hydrochloride of intermediate A. After isolation of desirable cis isomer, the product is further reacted with chloroacetyl chloride and then with methylamine in THF to give tadalafil.

Low solubility of tadalafil in aqueous solutions is further disadvantageous because in vivo absorption is typically dissolution rate-limited which may result in poor bioavailability of the drug. Different approaches in the processes of preparation of pharmaceutical compositions have been applied to overcome the poor solubility.

For example, EP 1 200 092 B1 describes a pharmaceutical composition of free drug particulate form of tadalafil wherein at least 90% of the particles have a particle size of less than about 40 µm as well as composition comprising tadalafil, wherein the compound is present as solid particles not embedded in polymeric co-precipitate. Apparently, preferably at least 90% of the particles have a particle size of less than 10 µm. The technological drawback of such small particles is possible chargeability and secondary agglomeration due to increased surface energy which can cause problems during the micronization and further processing.

WO 2008/134557 describes another approach to overcome the low-solubility problem by pharmaceutical composition comprising starch and tadalafil characterized by particle size having d(90) greater than 40 µm wherein the weight ratio of starch to tadalafil is 4.5 to 1 or greater. Apparently, the preferred ratio is at least 15 to 1.

Yet another approach to overcome the low-solubility problem is to use a "co-precipitate" of tadalafil and a carrier or excipient. For example, EP 828 479 B1 describes a solvent based process wherein tadalafil and a carrier are co-precipitated with a medium in which the tadalafil and carrier are substantially insoluble. EP 828 479 describes a solvent based process wherein tadalafil and hydroxypropyl methylcellulose phthalate are co-precipitated in weakly acidic medium from a combination of non-aqueous water miscible solvent and water. However, pharmaceutical composition prepared according to EP 828 479 exhibit deviations in release rate of tadalafil which was due to poor reproducibility of a process for preparation of co-precipitate. It was found that precipitation in acidic media causes unwanted degradation of hydroxypropyl methylcellulose phthalate and that precipitation at higher temperatures does not produce desired product.

WO 2008/005039 also describes a solid composite including tadalafil being in intimate contact with a carrier. The carriers include hydrophilic polymers such as povidone, cellulose derivatives, polyethylene glycol and polymethacrylates. The compositions are prepared by combining tadalafil with hydrophilic polymer and removal of the solvent by evaporation.

WO 2010/115886 describes an adsorbate comprising poorly soluble active ingredient with a particulate and/or porous carrier wherein the adsorbate is prepared by using non-polar solvent.

Apparently, the solvents used are selected from the group of chlorinated hydrocarbon (dichloromethane or trichloromethane), diisopropylether and hexane, which is also the main drawback of this solution.

Co-precipitates of phosphodiesterase-5-inhibitor and copolymer of different acrylic acid derivatives are described in WO 2011/012217. The procedures described involve the use of tetrahydrofurane.

Poor solubility can also be solved with co-crystals. WO 2010/099323 discloses crystalline molecular complexes of tadalafil with co-former selected from the group of a short to medium chain organic acids, alcohols and amines.

WO 2012/107541 and WO 2012/107092 disclose co-granulate of tadalafil with cyclodextrines.

WO 2014/003677 discloses a pharmaceutical composition comprising solid dispersion particles containing tadalafil and a dispersing component, which composition further comprises a solubilizer.

WO 96/38131 and patent family member EP 0 828 479 disclose solid dispersions of poorly soluble drugs, processes for their preparation and their use in pharmaceutical compositions. Specifically, solid dispersions of (6R,12aR)-2,3,6,7,12,12a-hexahydro-2-methy1-6-(3,4-methy1enedioxyphenyl)-pyrazino[2',I':6,I]pyrido[3,4-b]indole-1,4-dione and (+)-N-[I-(adamantanmethyl)-2,4-dioxo-5-phenyl-2,3,4,5-tet1ahydro-lH-1,5benzodiazepin-3-yl]-N'-phenylurea are described.

US 2012/0189694 A relates to a co-precipitate comprising a phosphodiesterase-5 inhibitor (PDE-5-inhibitor) and a pharmaceutically compatible copolymer carrier comprising 2 or more different acrylic acid derivatives, a method for production thereof and a medication comprising the co-precipitate, a method for producing said medication and the use of said medication for treating an illness wherein the inhibiting of phosphodiesterase-5 is of therapeutic benefit.

Based on the above, there is still a need for an improved dosage form containing tadalafil and improved technological process for the preparation thereof.

### Summary of the invention

The above problem is solved by the manufacturing process for preparation of co-precipitates of tadalafil as characterized in appended claim 1. Preferred embodiments of this aspect of the present invention are specified in appended claims 2 to 7. The resulting co-precipitated product is another aspect of the present invention, which is characterized in appended claim 8. Finally, the present invention solves the above problem by providing pharmaceutical compositions as characterized in appended claims 9 to 14.

### Figures

Figure 1 shows dissolution profiles of pharmaceutical composition prepared according to Examples F1-F2 and Comparative Examples F5-F9 in 0.1M HCI
Figure 2 shows storage time dependence of dissolution profile of pharmaceutical composition prepared according to Example F1 and Comparative Example F10

### DESCRIPTION OF THE INVENTION

The present invention provides a solid pharmaceutical composition comprising solid particles - co-precipitates, where tadalafil is in the intimate contact with a pharmaceutically acceptable polymer and which further comprises at least one pharmaceutically acceptable excipient essential to achieve an improved dissolution of tadalafil from solid pharmaceutical compositions, and a process for production thereof.

The present invention further provides a process for preparation of the solid particles - co-precipitates, as well as a simplified manufacturing process such as direct compression for preparation of the said solid pharmaceutical compositions.

One aspect of the invention is a process for preparation of co-precipitates of tadalafil with pharmaceutically acceptable excipients. In the present invention we have surprisingly discovered that in-vitro dissolution rate of co-precipitated tadalafil can be influenced by careful control of the co-precipitation process, namely by careful control of the temperature of the precipitation media. We have found out that co-precipitate when exposed to elevated temperature is affected with substantial growth of crystalline tadalafil particles.

A process for preparation of co-precipitates of tadalafil with pharmaceutically acceptable excipients comprises the steps a) to d) as specified in appended claim 1.

Pharmaceutically acceptable excipients, which can be used in step a), may be selected from but not limited to hydroxypropyl methylcellulose phthalate (HPMCP), hydroxypropyl methylcellulose acetate succinate, polyvinyl acetate phthalate. Preferably hydroxypropyl methylcellulose phthalate, such as types HP-50 or HP-55, is used. The weight ratio of tadalafil to pharmaceutically acceptable excipient may vary from 1:5 to 5:1, preferably from 1:2 to 2:1, most preferably the weight ratio is 1:1.

Any reaction vessel can be used provided that it can withstand the pressure chosen for step (b). For instance, a standard overpressure reactor, typically usable to pressures up to 0.6 MPa, can be used.

Typical addition times in step (b) may range from 5 min to 10 h and preferably from 30 min to 5 h. According to further embodiments of the present invention, addition times may range from 10 min to 1 h and preferably 20 min to 45 min or from 30 min to 3 h or more preferably 1 h to 2 h.

A preferred process for preparation of co-precipitates of tadalafil with pharmaceutically acceptable excipients comprises the following steps:
a) dissolving tadalafil and hydroxypropyl methylcellulose phthalate and optionally at least one further pharmaceutically acceptable excipients in organic solvent or mixture thereof or a mixture of the organic solvent or organic solvent mixture with water at elevated temperature and/or pressure,
b) adding the solution obtained in step a) into precipitation media at temperature from 0°C to 40°C, preferably from 0°C to 20°C, more preferably from 0°C to 10°C, most preferably from 0°C to 5°C and at pressure from normal atmospheric pressure to pressure of 0.6MPa, preferably at normal atmospheric pressure to 0.4MPa
c) optionally, the suspension formed under step b) may be heated to reflux temperature and the solvents may be at least partially distilled off,
d) isolating the obtained co-precipitate.

Organic solvents used in step a), may be selected from but are not limited to the group of solvents comprising ketones, alcohols, ethers or a mixture thereof, preferably the solvent is selected from the group comprising acetone, methanol and tetrahydrofuran, most preferably acetone and methanol are used. Preferably the mixture of solvents or the mixture of a solvent with water is used. It is a particularly preferred embodiment to use a mixture of methanol with at least one other organic solvent specified above, especially a mixture of methanol with acetone. It is another particularly preferred embodiment to use a mixture of an organic solvent with water and especially a mixture of acetone with water. The volume ratio of the solvents in the mixture, and especially the ratio of organic solvent:water and the ratio of other organic solvent:methanol, may vary from 1:1 to 15:1. Most preferably the mixture of acetone and water in the ratio of 5:1 to 10:1 (the minor component being water) and the mixture of acetone and methanol in the ratio of 1:1 to 5:1 (the minor component being methanol) is used.

The elevated temperature in step a) may vary from room temperature to the boiling point of the solvent mixture i.e. the reflux temperature, preferably the temperature in step a) is from 25°C to the boiling point of the solvent mixture, and the pressure may vary from atmospheric pressure to 0.6MPa, preferably to 0.4MPa. In one embodiment, "atmospheric pressure" or "normal atmospheric pressure", as used in the present application, is intended to characterize a pressure of 1013.25 hPa. The advantage of the present process is that quantity of solvents required to dissolve tadalafil can be reduced with applied pressure during the dissolving process, in step a) and during precipitation process in step b).

Precipitation media in step b) may be selected from polar protic solvents or non-polar aprotic solvents, such as water and C₅ to Cs alkanes, preferably water and heptane, most preferably water. In one preferred embodiment, water is used as the precipitation medium if the solvent mixture employed in step a) also includes water. In another preferred embodiment, a solvent or solvent mixture is used in step a), which does not contain water, if the precipitation medium employed in step b) is a C₅ to Cs alkane such as heptane. Yet another embodiment concerns the use of a solvent or solvent mixture in step a), which does not contain water, if the precipitation medium employed in step b) is water. Yet last embodiment concerns the use of solvent or solvent mixture in step a) which contains water, if the precipitation media is a C₅ to Cs alkane such as heptane. We have surprisingly found out that optimal results have been achieved when water at temperatures below 20°C, preferably below 10°C, most preferably below 5°C has been used as a precipitation media.

In case hydroxypropyl methylcellulose phthalate is used as pharmaceutically acceptable ingredient for preparation of co-precipitate we have discovered that it is favorable to use pure water as a precipitation media that is in contrast to prior art teaching that weakly acidic medium should be used. As hydroxypropyl methylcellulose phthalate is soluble at pH values above 5, it was not expected that it would co-precipitate with tadalafil in pure water, but it was surprisingly found out that co-precipitate free of crystalline particles of tadalafil is provided whereas in acidic media and at higher temperatures co-precipitate with substantial amounts of crystalline particles of tadalafil is formed.

We further discovered that by precipitating in non-acidic medium unwanted degradation of hydroxypropyl methylcellulose phthalate can be reduced in comparison to precipitation in weakly acidic medium. In case of precipitation in acidic medium a substantial amounts of phthalic acid is formed as a by-product which results in unacceptable product for incorporating into pharmaceutical formulation. Moreover, in the precipitate prepared by the process of the present invention the whole amount of tadalafil is completely co-precipitated with hydroxypropyl methylcellulose phthalate whereas processes known from prior art result in incomplete co-precipitation meaning that some particles of tadalafil still exist as free particles which are not in intimate contact with pharmaceutical excipient. It is thus a preferred embodiment of the present invention to carry out co-precipitation using water having a pH in the range of >5 to 7.5, more preferably 5.5 to 7.3 and most preferably 6 to 7.1. However, it is also possibly to carry out this step at a lower pH, e.g., in the range of from 1 to 5.

The organic solvent in optional step c) may be partially or completely evaporated in order to improve filtration properties of co-precipitate.

The co-precipitate formed is isolated by means of conventional isolation processes such as centrifugation, decanter centrifugation, pressure filtration, vacuum filtration. The obtained material can be dried in a dryer such as vacuum drier or fluid bed drier at temperatures from 40°C to 120°C, preferably from 40°C to 90°C. The dried co-precipitate may be further subjected to grinding, milling or micronisation if required.

Co-precipitate provided by the process according to the present invention is characterized by physical properties suitable for handling and processing by common industrial facilities.

For the milling purposes fluid energy mill, jet mill, ball mill, roller mill, pin mill or hammer mill can be used as milling equipment.

The term "average particle size" as used herein refers to the volume mean diameter of particles. The diameter and volume mean diameter can be determined by laser light scattering using preferably a Malvern-Mastersizer Apparatus MS 2000 and a Scirocco dispersion cell (dry dispersion).

The milled co-precipitate of the present invention is characterized by average particle size between 1 and 200 µm, preferably 5-100 µm, most preferably 10-60 µm.

Specific surface area of co-precipitate according to the present invention is more than 6 m²/g, preferably more than 10 m²/g and most preferably more than 15 m²/g. It is well known in the art, that for example micronized material with larger surface area is difficult to handle because of triboelectric effects and secondary agglomeration. Co-precipitate according to the present invention having high specific surface area does not show mentioned disadvantages. Moreover, when incorporated into the pharmaceutical composition according to present invention it shows desirable dissolution rates comparable to dissolution profile of reference drug product of tadalafil, namely CIALIS^{®}.

In the following table the comparison between properties of the free tadalafil particles, co-precipitate obtained by precipitation at low temperature and co-precipitate obtained by precipitation at high temperature is shown:

| Sample | Average particle size [µm] | Specific surface area [1 m²/g] | Processability |
|---|---|---|---|
| Low temperature co-precipitate (T≤40°C) | 30 to 60 | 10 to 50 | Excellent |
| High temperature co-precipitate (T>40°C) | 30 to 60 | 1 to 6 | Good |
| Tadalafil | 1 to 10 | | Poor |

The results show that the optimal material for use in the process for preparation of pharmaceutical composition is obtained by the process of the present invention, namely by co-precipitation at low temperature.

Tadalafil used as a starting material in the process of co-precipitation can be in any known crystalline or amorphous form of tadalafil, including solvates and hydrates.

The processes of co-precipitation can be performed in a special embodiment of the present invention as the last step of a tadalafil synthesis. It is a non-limiting preferred embodiment of the present invention to carry out the tadalafil synthesis of the present invention as described in the parent patent EP 3 172 207 B, followed by the co-precipitation method of the present invention.

Another aspect of the present invention is a pharmaceutical composition comprising tadalafil co-precipitate and at least one water soluble diluent and/or water insoluble non-swellable diluent, wherein the composition is substantially free of water insoluble swellable diluents, as specified in appended claim 9.

The term "substantially free" means that pharmaceutical composition comprises less than 5% wt. by tablet weight of water insoluble swellable diluents. In a preferred embodiment, these maximum amounts take into account all components that may be classified as water insoluble swellable diluents even if they are added for a different reason, e.g. for co-precipitation of tadalafil or as disintegrant or binder.

The content of water soluble diluent and/or water insoluble non-swellable diluent may vary from 50% to 95% wt. per tablet weight, preferably 55% to 90% wt. per tablet weight, most preferably 60% to 85% wt. per tablet weight.

The amount of tadalafil co-precipitate may vary from 2% to 20% wt. by tablet weight, preferably 5% to 15% wt. by tablet weight.

Surprisingly it has been found that presence of water soluble diluents or water insoluble non-swellable diluent and absence of water insoluble swellable diluents in tablet formulations comprising tadalafil co-precipitate are essential to achieve an improved dissolution profile of tadalafil as well as for the use of a simplified manufacture process such as direct compression instead of granulation for preparation the solid pharmaceutical compositions comprising the tadalafil co-precipitate according to the invention.

Solubility of the diluent is determined in terms of the number of parts by volume of water required to dissolve one part by weight of a solid or one part by volume of a liquid.

In statements of solubility according to the present invention, the terms used have the following significance, referred to a temperature between 15°C and 25°C.

| **Descriptive term** | **Approximate volume of solvent in millilitres per gram of solute** | | | |
|---|---|---|---|---|
| Very soluble | less than | 1 | | |
| Freely soluble | from | 1 | to | 10 |
| Soluble | from | 10 | to | 30 |
| Sparingly soluble | from | 30 | to | 100 |
| Slightly soluble | from | 100 | to | 1 000 |
| Very slightly soluble | from | 1 000 | to | 10 000 |
| Practically insoluble | more than | | | 10 000 |

As used herein, the term "water soluble" means that dissolving of 1g of excipient requires less than 30mL of water at temperature range between 15°C and 25°C, preferably less than 10 mL, most preferably less than 6 mL.

As used herein, the term "water insoluble" means that dissolving of 1g of excipient requires more than 30mL of water at temperature range between 15°C and 25°C, preferably more than 100 mL, most preferably more than 1000 mL.

In case of doubt, in one embodiment of the present invention, if the solubility of a substance at 15°C should differ significantly from its solubility at 25°C, the substance is to be classified as "water soluble" or "water insoluble" at the temperature of 20°C.

The water soluble diluent according to the present invention exhibits solubility which is characterized by requiring less than 30mL of water at temperature range between 15°C and 25°C, preferably less than 10 mL, most preferably less than 6 mL, for dissolving of 1g of the material.

Tadalafil co-precipitate suitable for incorporation into the pharmaceutical composition according to the present invention may be any co-precipitate prepared in accordance with cited prior art documents or co-precipitate prepared in accordance with the process disclosed in the present invention. Preferably co-precipitate prepared in accordance with the process disclosed in the present invention is used, most preferably co-precipitate of tadalafil with hydroxypropyl methylcellulose phthalate.

Water soluble diluents can be selected from but not limited to the group of carbohydrates such as dextrates; polysaccharides such as maltodextrin; monosaccharides such as glucose and fructose; oligosaccharides such as sucrose and lactose such as anhydrous lactose, amorphous lactose, spray-dried lactose, granulated lactose and lactose monohydrate, and sugar alcohols such as mannitol, erythritol, sorbitol, dulcitol, ribitol and xylitol.

The water insoluble diluent according to the present invention exhibits solubility which is characterized by requiring more than 30mL of water at temperature range between 15°C and 25°C, preferably more than 100 mL, most preferably more than 1000 mL.

Water insoluble non-swellable diluents can be selected from the group of calcium and magnesium anorganic salts such as anhydrous dibasic calcium phosphate, dibasic calcium phosphate dihydrate, magnesium oxide and calcium sulphate.

Water insoluble swellable diluents can be selected from the group of starches such as maize starch, potato starch, wheat starch and partially pregelatinized starches; cellulose and its derivatives such as microcrystalline cellulose, silicified microcrystalline cellulose and powdered cellulose. According to one embodiment of the present invention, the composition is substantially free of water insoluble further components selected from the group comprising starches such as maize starch, potato starch, wheat starch and partially pregelatinized starches; cellulose and its derivatives such as microcrystalline cellulose, silicified microcrystalline cellulose and powdered cellulose.

As water soluble diluents the use of sugar alcohols such as mannitol and/or saccharides such as sucrose and lactose is preferred, most preferably mannitol is used. As water insoluble non-swellable diluent dibasic calcium phosphate is preferably used.

It has been found out that the presence of water insoluble swellable diluent substantially influences the dissolution profile of tadalafil; therefore the content of water insoluble swellable diluents in the pharmaceutical composition should be kept as low as possible. By way of examples and comparative examples it was shown that presence of water insoluble swellable diluent such as microcrystalline cellulose in an amount above 20% wt. per tablet weight decreases the dissolution rate at any point in time by more than 10%.

In a preferred embodiment of the present invention a pharmaceutical composition comprises tadalafil co-precipitate and mannitol as a water soluble diluent, wherein the composition is substantially free of water insoluble swellable diluents.

In another preferred embodiment of the present invention a pharmaceutical composition comprising tadalafil co-precipitate and mannitol as a water soluble diluent, wherein the composition is substantially free of microcrystalline cellulose and preferably completely free of microcrystalline cellulose.

A pharmaceutical composition comprising tadalafil co-precipitate and at least one water soluble diluent and/or water insoluble non-swellable diluent wherein the composition is substantially free of water insoluble swellable diluents may further comprise one or more excipients selected from but not limited to the group of disintegrants, binders, lubricants, glidants and/or surfactants.

Disintegrant/-s may be selected from but not limited to the group of excipients that contains croscarmellose sodium, carmellose calcium, starch, pregelatinized starch and starch derivative such as sodium starch glycolate and/or crosslinked starch, crospovidone, low-substituted hydroxypropyl cellulose and polacrilin potassium, most preferably croscarmellose sodium. The amount of the disintegrant used in the composition according to present invention may vary from 0 to 10% by tablet weight, preferably less than 5% by tablet weight.

Binder/-s may be selected from but not limited to the group of excipients that contains polyvinylpyrrolidone, polyvinylpyrrolidone-vinyl acetate copolymer, low-substituted hydroxypropyl cellulose, hydroxypropyl cellulose and hydroxypropyl methylcellulose, preferred binder is polyvinylpyrrolidone or polyvinylpyrrolidone-vinyl acetate copolymer. The amount of binder used in the composition according to present invention may vary from 0 to 10% by tablet weight, preferably less than 5%, more preferably the composition is binder free.

Lubricant/-s may be selected from but not limited to the group of excipients that contains sodium stearyl fumarate, magnesium stearate, stearic acid, glyceryl behenate and talc, preferably magnesium stearate is used. The amount of lubricant may vary from 0.1 to 5% by tablet weight, preferably from 0.1 to 2% by tablet weight is used.

Glidant/-s may be selected from but not limited to the group of excipients that contains colloidal silicon dioxide, hydrophobic colloidal silica and talc. The amount of glidant may vary from 0% to 5% by tablet weight; preferably from 0 to 2% by tablet weight is used.

Surfactant/-s may be selected from but not limited to the group of excipients that contains sodium lauryl sulphate and polysorbate. Sodium lauryl sulphate is preferably used. The amount of surfactant may vary from 0% to 10% by tablet weight, preferably from 0 to 5% by tablet weight.

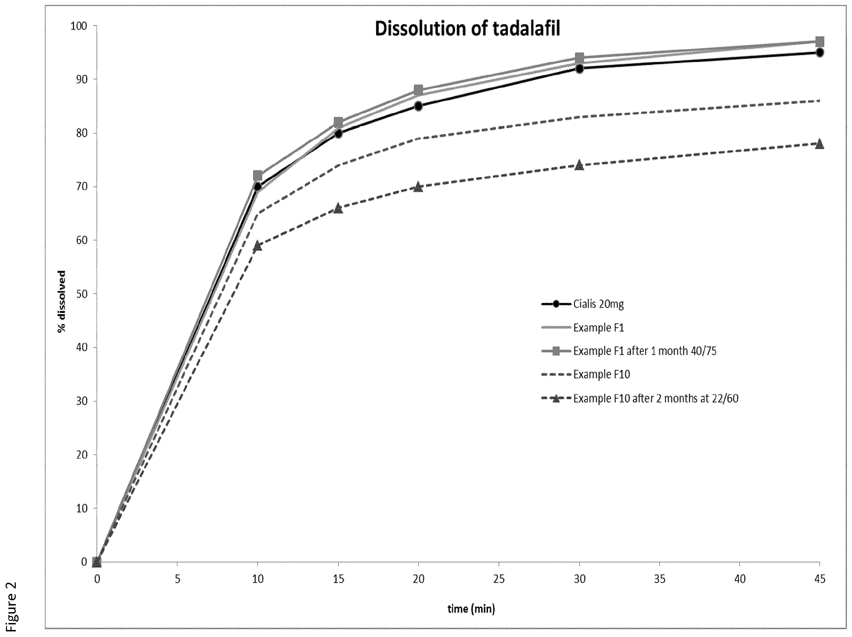

Another advantage of the pharmaceutical composition of the present invention is storage stability in terms of invariability of the dissolution profile during storage time. The composition of the present invention is characterized in that the release profiles of tadalafil do not change upon long-term storage, whereas as demonstrated by way of Example F10 the composition comprising more than 20% by tablet weight of microcrystalline cellulose exhibits storage time depended dissolution profile as shown in Figure 2.

According to the invention, "storage stable" or "time stable" also means that after storage under standard conditions the active compounds show release profiles as they would upon immediate use without storage. According to the invention, the admissible fluctuations with respect to the release profile are characterized in that the amount released per time unit fluctuates by no more than 10%, preferably no more than 7% and especially preferably no more than 5 %, with respect to a mean value.

The mean value is calculated from six measurements of the release profile.

Preferably, the release of the active compounds from a storage stable formulation is determined by testing samples containing 20 mg of tadalafil in 900mL of an aqueous solution of 0.1M HCI + 0.2% sodium lauryl sulfate at 37°C, using USP Apparatus I with baskets rotating at a speed of 100 rpm and automatic UV detection.

The pharmaceutical compositions of the present invention are solid pharmaceutical compositions. The compositions can be in the form of tablets, minitablets, microtablets, coated tablets, coated minitablets, coated microtablets, pills, lozenges, etc. The pharmaceutical compositions of the present invention are preferably suitable for oral application. The formulations of the present invention preferably have the form of tablets. The term "tablet" as used herein is intended to encompass compressed pharmaceutical dosage formulations of all shapes and sizes. Coated tablets are particularly preferred.

The pharmaceutical compositions of the present invention are preferably formulated in a unit dosage form, each dosage form containing from about 0.1 to about 100 mg of tadalafil, preferably from about 1 mg to about 40 mg of tadalafil, more preferably 2.5 mg, 5 mg, 10mg or 20 mg of tadalafil. The amounts given are calculated on the content of tadalafil.

According to a preferred embodiment of the present disclosure the tablets of the present invention are prepared by dry method such as direct compression or dry granulation, preferably by direct compression.

The manufacturing process for preparation of the pharmaceutical composition according to the present invention comprises mixing the co-precipitate with diluent/-s and optionally with other excipient/-s and formulating the mixture into a solid dosage form such as capsule or tablet, where tablet is being preferred option. Dosage forms comprising co-precipitate can be formulated using any of the dry formulation processes that are known to the one skilled in the art. Preferably direct compression of the co-prepicitate admixed with at least one water soluble diluent and optionally further excipients selected from other diluents, disintegrants, surfactants, glidants, lubricants and optionally other excipients is performed. Direct compression of the mixture comprises dividing the mixture into unit doses that comprise a therapeutic amount of tadalafil and compressing the unit doses into tablets with a tablet press.

According to the present disclosure there is provided a direct compression method for manufacturing the pharmaceutical formulation which comprises the following steps:
- mixing tadalafil co-precipitate and at least one water soluble and/or water insoluble non-swellable diluent and optionally a mixture of further excipients to form a powder mixture which can be optionally sieved before mixing to give compression mixture;
- compressing the compression mixture to desired solid dosage form;
- optionally, applying a film coating.

In yet another surprising discovery we have found that by the use of water soluble diluents and/or water insoluble non-swellable diluents in the process of preparation of solid composition, compaction force insensitivity of dissolution profile of solid dosage form comprising tadalafil is achieved. Therefore in the manufacturing steps prior to final compression dry granulation of ingredients such as roller compaction or the slugging of the co-precipitate admixed with one or more water soluble diluents and/or water insoluble non-swellable diluents can be performed to improve the flowability of the compression mixture and/or homogeneity of the final dosage form. Roller compaction typically comprises a process in which powders are processed into densified sheets by the use of mechanical pressure exerted on two compacting rolls. The densified sheets are milled to any desired mesh size.

According to the present disclosure there is provided a dry granulation method for manufacturing the pharmaceutical formulation which comprises:
- dry granulating optionally sieved tadalafil co-precipitate and water soluble diluent and/or water insoluble non-swellable diluents and optionally at least one excipient or a mixture of excipients using slugging and/or roller compaction;
- optionally milling and sieving the obtained slugs or compacts to obtain granulate with desired particle size distribution;
- optionally addition of excipient or a mixture of excipients to the granulate to give compression mixture;
- compressing the compression mixture to the desired solid dosage form;
- optionally, applying a film coating.

It is preferred that tadalafil co-precipitate containing tablets are further film-coated. Film-coating is present in the range from 1% to 10% by weight of the total amount of solid medical preparation, preferably between 1% and 5% by weight. The thickness of the coating is in the range of 2-40 µm, preferably 3-30 µm, most preferably 3-25 µm, measured on the cross-section of film-coated tablet using scanning electron microscope. In one embodiment of the invention film-coating is water soluble and is based on soluble cellulose ethers such as hypromellose (hydroxypropyl methylcellulose) or hyprolose (hydroxypropyl cellulose). In another embodiment of the invention film-coatings having reduced permeation for gases such as moisture and/or oxygen can be applied onto the tablet cores. Such coatings are based on polymers, which can be selected from polyvinyl alcohol, block copolymer of polyvinyl alcohol and polyethylene glycol (e.g., Kollicoat^{®} IR and Kollicoat^{®} Protect), derivatives of polymethacrylates such as Eudragit^{®} E, carboxymethyl cellulose and other similar film-coatings. Further, additives selected form pharmaceutically acceptable plasticizers, colorants, pigments and antitacking agents can be included in the coating in order to achieve optimal coating characteristics. Plasticizers can be selected from polyethylene glycol having average molecular weight of 200 to 8,000, propylene glycol, esters of citric acid such as triacetyl citrate, butyl sebacate etc. Pigments can be selected from metal oxides such as titanium and iron oxides or mixture thereof. Antitacking agents can be selected from talc, colloidal silicon dioxide, glycerol monostearate, magnesium stearate and the like.

The mixture can also be filled into the hard gelatin or HPMC capsule shells or soft gelatin capsules.

Another aspect of the present invention within the scope of appended claim 9 is an orally disintegrating pharmaceutical composition comprising tadalafil co-precipitate and at least one water soluble diluent, wherein the composition is substantially free of water insoluble swellable diluents such that the content of water insoluble swellable diluents is less than 5 wt.% by tablet weight. The composition disintegrates or dissolves in oral cavity in the presence of small amounts of water or physiological fluid such as saliva. Disintegration time of said dosage form according to Ph. Eur. 8th Ed. is less than 3 minutes in water, preferably less than 1 minute.

Water soluble diluents used in the said dosage form can be selected from but not limited to the group of carbohydrates such as dextrates; polysaccharides like maltodextrin; monosaccharides such as glucose and fructose; oligosaccharides such as sucrose and lactose, such as anhydrous lactose, amorphous lactose, spray-dried lactose, granulated lactose and lactose monohydrate and sugar alcohols such as mannitol, erythritol, sorbitol, dulcitol, ribitol and xylitol.

The composition is substantially free of water insoluble swellable diluents selected from the group of starches such as maize starch, potato starch, wheat starch and partially pregelatinized starches; cellulose and its derivatives such as microcrystalline cellulose, silicified microcrystalline cellulose and powdered cellulose.

In particular, the orally disintegrating pharmaceutical composition according to the invention as specified in the appended claims comprises
(a) tadalafil co-precipitate,
(b) mannitol, and
(c) calcium silicate.

The composition comprises preferably 40% to 90%, more preferably 50% to 85% and most preferred 60% to 80% by weight of mannitol.

The component (c) of the composition is calcium silicate. The calcium silicate can be in crystalline or amorphous form or a mixture thereof. The particle size of the calcium silicate is preferably in the range from 1 to 500 µm. The average particle size is preferably 1 to 100 µm. The composition comprises preferably 5% to 40%, more preferably 10% to 30% and most preferably 12% to 25% by weight of calcium silicate.

Moreover, the composition usually comprises at least one further excipient, other than mannitol and calcium silicate, selected from the group of disintegrants, diluents, flavouring agents, sweetening agents, glidants, colouring agents and lubricants.

The disintegrant is preferably selected from the group consisting of crospovidone, croscarmellose sodium, sodium starch glycolate and low-substituted hydroxypropyl cellulose. Preferred disintegrants are crospovidone and low substituted hydroxypropyl cellulose, more preferably a combination of both. Low-substituted hydroxypropyl cellulose has preferably a content of hydroxypropyl groups of 7.0% to 12.9% and in particular 10.0% to 12.9% by weight. Examples of useful low-substituted hydroxypropyl celluloses are available under the tradenames LH-11, LH-20, LH-21, LH-22, LH-30, LH-31 and LH-32 from Shin-Etsu Chemical Co. Ltd.

It is further preferred that the composition comprises 1% to 20%, more preferably 3% to 15% by weight of disintegrant.

As already mentioned, diluents used in the said dosage form are water soluble and can be selected from but not limited to the group of carbohydrates such dextrates; polysaccharides like maltodextrin; monosaccharides such as glucose and fructose; oligosaccharides such as sucrose and lactose, for example anhydrous lactose, amorphous lactose, spray-dried lactose, granulated lactose and lactose monohydrate and sugar alcohols such as mannitol, erythritol, sorbitol, dulcitol, ribitol and xylitol.

Suitable sweeteners include sugars, such as sucrose, lactose and glucose; cyclamate and salts thereof; saccharin and salts thereof; and aspartame. The preferred sweetener is aspartame.

Flavouring agents can be natural or synthetic flavours such as strawberry flavour, wild cherry flavour, green apple flavour, spearmint flavour, and peppermint flavour.

Suitable lubricants are magnesium stearate, stearic acid, sodium stearyl fumarate, magnesium lauryl sulphate, polyethylene glycol, and glyceryl behenate. The preferred lubricants are magnesium stearate and sodium stearyl fumarate.

The selected examples illustrate the present invention without limiting the scope of the invention.

### EXAMPLES

### Methods

The chemical purity of tadalafil is assessed by high pressure liquid chromatography (HPLC) with a column of the type Zorbax SB-C8 250 x 4.6 mm i.d., 5 µm particles; column temperature: 40°C; detector: UV 220 nm; flow rate: 0.7 ml/min; injection volume: 5 µL; mobile phase: A: 0.01M phosphate buffer solution pH 2; B: acetonitrile/methanol = 80/20 (V/V); Gradient (applicable for analysis of chromatographic purity): 0'=15% B, 7.5'=40% B, 14'=60% B, 20'-22'=90% B, 23'-28'=95% B, 30'-33'=15% B; Isocratic separation (applicable for analysis of assay): 40% of mobile phase A and 60% of mobile phase B.

This HPLC method is generally applicable for the analysis of tadalafil, chromatographic purity and assay.

### Sample preparation:

Chromatographic purity: The sample solution is prepared in concentration about 0.25 mg/mL. Sonication is applied during dissolving.

Dilution solvent is 90% methanol.

Assay: The sample solution is prepared in concentration of about 0.1 mg/mL. Sonication is applied during dissolving.

### Calculation:

Chromatographic purity: Area per cent method was used, solvent peaks were not integrated.

Assay: External standard method was used.

Unless otherwise specified, dissolution profiles were obtained by testing samples containing 20mg of tadalafil in 900mL of an aqueous solution of 0.1M HCI + 0.2% sodium lauryl sulfate at 37°C, using USP Apparatus I with baskets rotating at a speed of 100 rpm and automatic UV detection.

Unless otherwise specified, the tadalafil co-precipitate used in the examples has average particle size from 10 to 60 µm. The term "average particle size" as used herein refers to the volume mean diameter of particles. The diameter and volume mean diameter can be determined by laser light scattering using preferably a Malvern-Mastersizer Apparatus MS 2000 and a Scirocco dispersion cell (dry dispersion).

Tablet hardness was measured by determining lateral breaking strength using a KRAEMER (UTS) system.

Specific surface area of tadalafil and tadalafil co-precipitate s was determined by a gas sorption system based on the nitrogen adsorption, using the 6-point Brunauer, Emmett and Teller (BET) method. Prior to the analysis the sample was degassed for 2 hours under vacuum

### Comparative Example 1: Preparation of tadalafil co-precipitate with HPMCP HP-50, Precipitation at higher temperature

Tadalafil (100 g) and hydroxypropyl methylcellulose phthalate (100 g) were dissolved in a mixture of acetone (2430mL) and water (270mL) at reflux temperature. Solution was hot filtered and added to 0.25 M HCI in water (4150mL) at 65°C. Precipitate was collected by vacuum filtration, washed with water and dried in vacuum tray dryer up to 70°C. Dry material was milled by a pin mill. HPLC assay of tadalafil was 48.5%; average particle size of co-precipitate was 53 µm, specific surface area 2.5 m²/g.

### Example 1: Preparation of tadalafil co-precipitate with HPMCP HP-50

Tadalafil (1 kg) and hydroxypropyl methylcellulose phthalate (1 kg) were dissolved in mixture of acetone (20L) and water (3 L) at 54°C and under pressure 0.1MPa. Solution was hot filtered and added to water (42 L) at 2°C. Suspension was heated up to reflux and acetone was distilled off. Tadalafil co-precipitate was collected by pressure filtration and dried in vacuum dryer. Dry material was milled by a pin mill. HPLC assay of tadalafil was 53.5%.

### Example 2: Preparation of tadalafil co-precipitate with HPMCP HP-50

Tadalafil (1 kg) and hydroxypropyl methylcellulose phthalate (1 kg) were dissolved in mixture of acetone (20 L) and water (3 L) at 54°C and under pressure 0.1MPa. Solution was hot filtered and added to water (42 L) at 2°C. Suspension was heated up to reflux and acetone was distilled off. Tadalafil co-precipitate was collected by centrifuge and dried in a fluid bed dryer. Dry material was milled by a pin mill. HPLC assay of tadalafil was 52.5 %.

### Example 3: Preparation of tadalafil co-precipitate with HPMCP HP-50

Tadalafil (0.786 kg) and hydroxypropyl methylcellulose phthalate (1.140 kg) were dissolved in a mixture of acetone (24L) and water (2.3 L) at 54°C and under pressure 0.1MPa. Solution was filtered hot and added to water (42 L) at 2°C. Suspension was collected by centrifuge and dried in a vacuum tray dryer up to 70°C. Dry material was milled by a pin mill. HPLC assay of tadalafil was 43.5 %, average particle size of co-precipitate was 49 µm, specific surface area 31.0 m²/g.

### Example 4: Preparation of tadalafil co-precipitate with HPMCP HP-50

Tadalafil (2 g) and hydroxypropyl methylcellulose phthalate HP 50 (2 g) were dissolved in a mixture of acetone (48.5mL) and water (5.5mL) at reflux temperature. To obtained solution crospovidone (1g) was added. Obtained suspension was co-precipitated in water (83mL) at 2°C. Obtained material was collected with a vacuum filter and dried in vacuum dryer up to 90°C. HPLC assay of tadalafil 39.9%. Yield was 90%.

### Example 5: Preparation of tadalafil co-precipitate with HPMCP HP-50

Tadalafil (2 g) and hydroxypropyl methylcellulose phthalate HP 50 (2 g) were dissolved in a mixture of acetone (54mL) and methanol (19mL) at reflux temperature. To obtained solution crospovidone (1g) was added. Obtained suspension was co-precipitated in heptane (83mL) at 0°C. Obtained material was collected with a vacuum filter and dried in vacuum dryer up to 50°C. HPLC assay of tadalafil was 36.1 %. Yield was 90%.

### Example 6: Preparation of tadalafil co-precipitate with HPMCP HP-50

Tadalafil (2 g) and hydroxypropyl methylcellulose phthalate HP 50 (2 g) were dissolved in a mixture of aceton (54mL) and methanol (19mL) at reflux temperature. Obtained solution was co-precipitated in heptane (83mL) at 0°C. Obtained material was collected with a vacuum filter and dried in vacuum dryer up to 50°C. HPLC assay of tadalafil was 36.1 %. Yield was 90%.

### Example 7: Preparation of tadalafil co-precipitate with HPMCP HP-50

Tadalafil (1.3 kg) and hydroxypropyl methylcellulose phthalate (1.53 kg) were dissolved in mixture of acetone (32 L) and water (4 L) at 54°C and 1000 mbar. Solution was hot filtered and added to water (54 L) at 2°C. Tadalafil co-precipitate was collected by decanter centrifuge and dried in a vacuum drier. Dry material (2.4kg) was milled in a pin mill. HPLC assay of tadalafil was 48.8 %; average particle size of co-precipitate was 54 µm and specific surface area 26.1 m²/g.

### Example 8: Preparation of tadalafil co-precipitate with hydroxypropyl cellulose

Tadalafil (3g) and Klucel ELF (3g) was dissolved in a mixture of acetone (73mL) and water (8mL) at 50°C. Solution was hot filtered and added to 125mL water at 90°C. After that acetone was distilled off at 65°C and suspension was stirred for additional hour. Precipitated material was filtered using preheated filter funnel and dried at 80°C. Yield 3.8 g, HPLC assay was 50.0%.

### Example 9: Preparation of tadalafil co-precipitate with hydroxypropyl cellulose

Tadalafil (3g) and Klucel ELF (3g) was dissolved in a mixture of acetone (73mL) and water (8mL) at 50°C. Solution was hot filtered and added to 125mL water at 90°C with dissolved lactose (14g) at 90°C. After that acetone was distilled off at 65°C and suspension was stirred for additional hour. Precipitated material was filtered using preheated filter funnel and dried at 80°C. Yield 5 g, HPLC assay was 48.8%.

### Examples of tablets prepared according to the present invention

Example F1: Tablets containing tadalafil co-precipitate with HPMCP HP-50 prepared in accordance with Example 11 with water soluble mannitol and without swellable water insoluble diluents

| Component | Amount (mg) |
|---|---|
| Tadalafil co-precipitate with HPMCP HP-50 | 42.3 |
| Mannitol (Parteck M200) | 427.7 |
| Croscarmellose sodium | 15.0 |
| Sodium lauryl sulphate | 10.0 |
| Magnesium stearate | 5.0 |

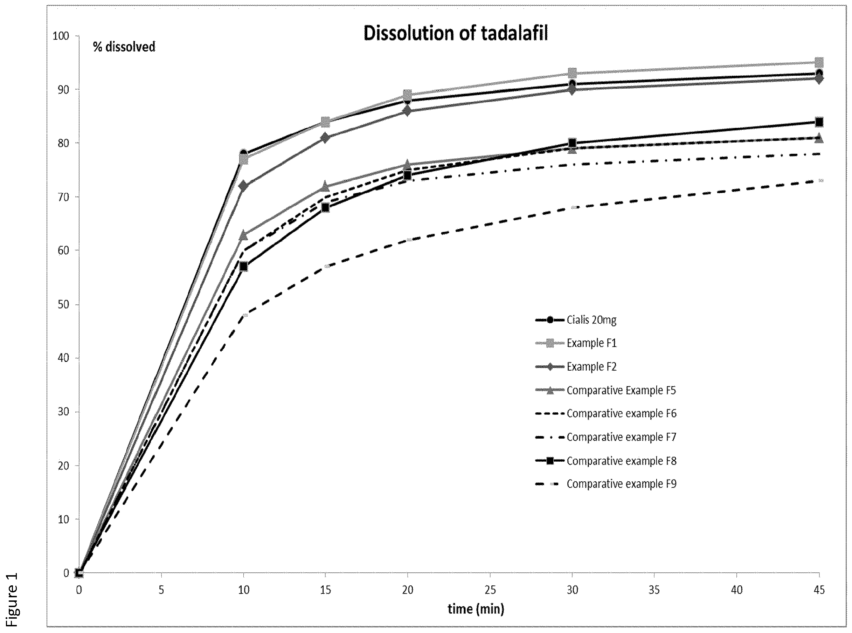

Tadalafil co-precipitate with HPMCP HP-50 was homogeneously mixed with mannitol, croscarmellose sodium and sodium lauryl sulphate. The magnesium stearate was added and mixed. The resultant blend was compressed into tablets. Dissolution profile of the example is shown in Figure 1.

Example F2: Tablets containing tadalafil co-precipitate with HPC prepared in accordance with Example 13 with water soluble mannitol and without swellable water insoluble diluents

| Component | Amount (mg) |
|---|---|
| Tadalafil co-precipitate with HPC | 40.0 |
| Mannitol (Parteck M200) | 430 |
| Croscarmellose sodium | 15.0 |
| Sodium lauryl sulphate | 10.0 |
| Magnesium stearate | 5.0 |

Tadalafil co-precipitate with HPC was homogeneously mixed with mannitol, croscarmellose sodium and sodium lauryl sulphate. The magnesium stearate was added and mixed. The resultant blend was compressed into tablets. Dissolution profile of the example is shown in Figure 1.

Example F3: Tablets containing tadalafil co-precipitate with HPMCP with water soluble spray-dried lactose and without swellable water insoluble diluents

| Component | Amount (mg) |
|---|---|
| Tadalafil co-precipitate with HPMCP HP-50 | 42.3 |
| Spray-dried lactose (Flowlac) | 407.7 |
| Pregelatinised starch 1500 | 35.0 |
| Sodium lauryl sulphate | 10.0 |
| Magnesium stearate | 5.0 |

Tadalafil co-precipitate with HPMCP was homogeneously mixed with spray-dried lactose, starch 1500 and sodium lauryl sulphate. The magnesium stearate was added and mixed. The resultant blend was compressed into tablets.

Example F4: Tablets containing tadalafil co-precipitate with HPMCP with water insoluble non-swellable anhydrous dibasic calcium phosphate and without swellable water insoluble diluents

| Component | Amount (mg) |
|---|---|
| Tadalafil co-precipitate with HPMCP HP-50 | 42.3 |
| Anhydrous dibasic calcium phosphate | 413.7 |
| Croscarmellose sodium | 30.0 |
| Sodium lauryl sulphate | 10.0 |
| Magnesium stearate | 5.0 |

Tadalafil co-precipitate with HPMCP was homogeneously mixed with calcium phosphate, croscarmellose sodium and sodium lauryl sulphate. The magnesium stearate was added and mixed. The resultant blend was compressed into tablets.

### Comparative examples of tablets containing microcrystalline cellulose

Comparative example F5: Tablets containing tadalafil co-precipitate with HPMCP HP-50 with water soluble mannitol and water insoluble swellable microcrystalline cellulose as diluent

| Component | Amount (mg) |
|---|---|
| Tadalafil co-precipitate with HPMCP HP-50 | 41.0 |
| Mannitol (Parteck M200) | 255.0 |
| Microcrystalline cellulose | 85.0 |
| Croscarmellose sodium | 12.1 |
| Sodium lauryl sulphate | 8.0 |
| Magnesium stearate | 2.0 |

Tadalafil co-precipitate with HPMCP HP-50 was homogeneously mixed with mannitol, microcrystalline cellulose, croscarmellose sodium and sodium lauryl sulphate. The magnesium stearate was added and mixed. The resultant blend was compressed into tablets. Dissolution profile of the example is shown in Figure 1.

Comparative example F6: Tablets containing tadalafil co-precipitate with HPMCP HP-50 with water soluble lactose anhydrous and water insoluble swellable microcrystalline cellulose as diluent

| Component | Amount (mg) |
|---|---|
| Tadalafil co-precipitate with HPMCP | 41.0 |
| Microcrystalline cellulose | 85.0 |
| Lactose anhydrous | 255.0 |
| Croscarmellose sodium | 12.1 |
| Sodium lauryl sulphate | 8.0 |
| Magnesium stearate | 2.0 |

Tadalafil co-precipitate with HPMCP HP-50 was homogeneously mixed with lactose anhydrous, microcrystalline cellulose, croscarmellose sodium and sodium lauryl sulphate. The magnesium stearate was added and mixed. The resultant blend was compressed into tablets. Dissolution profile of the example is shown in Figure 1.

Comparative example F7: Tablets containing tadalafil co-precipitate with HPMCP HP-50 with water soluble lactose monohydrate and spray dried lactose and water insoluble swellable microcrystalline cellulose as diluent

| Component | Amount (mg) |
|---|---|
| Tadalafil co-precipitate with HPMCP | 41.0 |
| Microcrystalline cellulose | 85.0 |
| Lactose monohydrate | 170.0 |
| Spray dried lactose | 85.0 |
| Croscarmellose sodium | 12.1 |
| Sodium lauryl sulphate | 8.0 |
| Magnesium stearate | 2.0 |

Tadalafil co-precipitate with HPMCP HP-50 was homogeneously mixed with lactose monohydrate, spray dried lactose, microcrystalline cellulose, croscarmellose sodium and sodium lauryl sulphate. The magnesium stearate was added and mixed. The resultant blend was compressed into tablets. Dissolution profile of the example is shown in Figure 1.

Comparative example F8: Tablets containing tadalafil co-precipitate with HPMCP HP-50 with water insoluble non-swellable calcium phosphate and water insoluble swellable microcrystalline cellulose as diluent

| Component | Amount (mg) |
|---|---|
| Tadalafil co-precipitate with HPMCP | 41.0 |
| Microcrystalline cellulose | 85.0 |
| Anhydrous dibasic calcium phosphate | 255.0 |
| Croscarmellose sodium | 12.1 |
| Sodium lauryl sulphate | 8.0 |
| Magnesium stearate | 2.0 |

Tadalafil co-precipitate with HPMCP HP-50 was homogeneously mixed with calcium phosphate, microcrystalline cellulose, croscarmellose sodium and sodium lauryl sulphate. The magnesium stearate was added and mixed. The resultant blend was compressed into tablets. Dissolution profile of the example is shown in Figure 1.

Comparative example F9: Tablets containing tadalafil co-precipitate with HPMCP HP-50 with only water insoluble swellable microcrystalline cellulose as diluent

| Component | Amount (mg) |
|---|---|
| Tadalafil co-precipitate with HPMCP | 41.0 |
| Microcrystalline cellulose | 340.0 |
| Croscarmellose sodium | 12.1 |
| Sodium lauryl sulphate | 8.0 |
| Magnesium stearate | 2.0 |

Tadalafil co-precipitate with HPMCP HP-50 was homogeneously mixed with microcrystalline cellulose, croscarmellose sodium and sodium lauryl sulphate. The magnesium stearate was added and mixed. The resultant blend was compressed into tablets. Dissolution profile of the example is shown in Figure 1.

Comparative example F10: Tablets containing tadalafil co-precipitate with HPMCP HP-50 with water insoluble swellable microcrystalline cellulose and cellactose as diluents

| Component | Amount (mg) |
|---|---|
| Tadalafil co-precipitate with HPMCP | 44.5 |
| Microcrystalline cellulose | 136.0 |
| Lactose monohydrate and powdered cellulose (Cellactose 80) | 195.5 |
| Croscarmellose sodium | 15.0 |
| Sodium lauryl sulphate | 8.0 |
| Magnesium stearate | 4.0 |

Tadalafil co-precipitate with HPMCP HP-50 was homogeneously mixed with microcrystalline cellulose, cellactose, croscarmellose sodium and sodium lauryl sulphate. The magnesium stearate was added and mixed. The resultant blend was compressed into tablets. Dissolution profile of the example F10 is shown in Figure 2, together with dissolution profiles of the same sample, taken after two months at 22°C and 60% RH.

In comparison, dissolution profile of composition according to invention is unaffected by storage at 40°C/75% for one month (Figure 2).

The aforementioned tablet formulations were film-coated with a film-coating dispersion containing:

| Component | Amount (mg) |
|---|---|
| Lactose monohydrate | 3 |
| Hypromellose | 3.95 |
| Talc | 2.4 |
| Triacetin | 0.85 |
| Titanium dioxide | 1.63 |
| Iron oxide, yellow | 0.67 |

Figures 1 and 2 show dissolution profiles of tablet formulations comprising tadalafil co-precipitates prepared according to listed examples. Dissolution conditions comprise: basket apparatus (USP I), 100 RPM, 0.1M HCI + 0.2% SDS, 900 mL

## Claims

1. A process for preparation of co-precipitates of tadalafil with pharmaceutically acceptable excipients which comprises the following steps:
a) dissolving tadalafil and at least one pharmaceutically acceptable excipient in an organic solvent or a mixture thereof or a mixture thereof with water at elevated temperature and/or pressure,
b) adding solution obtained in step a) into precipitation media at temperature from 0°C to 40°C, preferably from 0°C to 20°C, more preferably from 0°C to 10°C, most preferably from 0°C to 5°C and at pressure from normal atmospheric pressure to pressure of 0.6MPa, preferably at normal atmospheric pressure to 0.4MPa, or at a pressure within the range of from 0.11 MPa to 0.6 MPa and preferably 0.12 MPa to 0.6 MPa,
c) optionally, the suspension formed under step b) may be heated to reflux temperature and the solvents may be at least partially distilled off,
d) isolating the obtained co-precipitate.

2. A process according to claim 1, wherein pharmaceutically acceptable excipient used in step a), may be selected from hydroxypropyl methylcellulose phthalate (HPMCP), hydroxypropyl methylcellulose acetate succinate, polyvinyl acetate phthalate, preferably hydroxypropyl methylcellulose phthalate.

3. A process according to claims 1 and 2, wherein the solvent used in step a) is selected from the group of ketones, alcohols, ethers or a mixture thereof, or a mixture thereof with water.

4. A process according to claim 3, wherein the solvent used in step a) is a mixture of acetone with water or a mixture of acetone with methanol.

5. A process according to any of the claims 1 to 4, wherein precipitation media in step b) is selected from polar protic or non-polar aprotic solvents.

6. A process according to any of the claims 1 to 5, wherein the precipitation media in step b) is selected from water and heptane, preferably water.

7. A process according to any of the claims 1 to 6, wherein in step b) the precipitation media is water at temperature below 20°C, preferably below 10°C, most preferably below 5°C.

8. Co-precipitate obtainable by any of the claims 1 to 7, **characterized by** specific surface area of more than 6 m²/g, preferably more than 10 m²/g and most preferably more than 15 m²/g.

9. A pharmaceutical composition comprising tadalafil co-precipitate and at least one water soluble diluent and/or water insoluble non-swellable diluent, wherein the composition is substantially free of water insoluble swellable diluents such that the content of water insoluble swellable diluents is less than 5 wt.% by tablet weight.

10. A pharmaceutical composition according to claim 9, wherein the content of water soluble diluent and/or water insoluble non-swellable diluent may vary from 50% to 95% wt. per tablet weight, preferably 55% to 90% wt. per tablet weight, most preferably 60% to 85% wt. per tablet weight.

11. A pharmaceutical composition according to claims 9 or 10, wherein the content of water insoluble further component is less than 10% wt., most preferably less than 5% wt. by tablet weight.

12. A pharmaceutical composition according to claims 9 to 11, wherein water soluble diluent is selected from the group comprising carbohydrates such as dextrates; polysaccharides such as maltodextrin; monosaccharides such as glucose and fructose; oligosaccharides such as sucrose and lactose, such as anhydrous lactose, amorphous lactose, spray-dried lactose, granulated lactose and lactose monohydrate, and sugar alcohols such as mannitol, erythritol, sorbitol, dulcitol, ribitol and xylitol.

13. A pharmaceutical composition according to claims 9, 10 and 12, wherein the water insoluble swellable diluent is selected from the group comprising starches such as maize starch, potato starch, wheat starch and partially pregelatinized starches; cellulose and its derivatives such as microcrystalline cellulose, silicified microcrystalline cellulose and powdered cellulose.

14. A pharmaceutical composition according to claims 9 to 13, wherein the water soluble diluent is mannitol and wherein the composition is substantially free and preferably completely free of microcrystalline cellulose.

## Patentansprüche

1. Verfahren zur Herstellung von Co-Präzipitaten von Tadalafil mit pharmazeutisch akzeptablen Hilfsstoffen, das die folgenden Schritte umfasst:
a) Auflösen von Tadalafil und mindestens einem pharmazeutisch akzeptablen Hilfsstoff in einem organischen Lösungsmittel oder einer Mischung davon oder einer Mischung davon mit Wasser bei erhöhter Temperatur und/oder Druck,
b) Zugabe der in Schritt a) erhaltenen Lösung in ein Fällungsmedium bei einer Temperatur von 0 °C bis 40 °C, vorzugsweise von 0 °C bis 20 °C, noch bevorzugter von 0 °C bis 10 °C, am meisten bevorzugt von 0 °C bis 5 °C und bei einem Druck von normalem Luftdruck bis zu einem Druck von 0,6 MPa, vorzugsweise bei normalem Luftdruck bis 0,4 MPa oder bei einem Druck im Bereich von 0,11 MPa bis 0,6 MPa und vorzugsweise 0,12 MPa bis 0,6 MPa,
c) optional kann die in Schritt b) gebildete Suspension auf Rückflusstemperatur erwärmt werden und die Lösungsmittel können zumindest teilweise abdestilliert werden,
d) Isolierung des erhaltenen Co-Präzipitats.

2. Verfahren gemäß Anspruch 1, bei dem der in Schritt a) verwendete pharmazeutisch akzeptable Hilfsstoff, ausgewählt werden kann aus Hydroxypropylmethylcellulosephthalat (HPMCP), Hydroxypropylmethylcelluloseacetatsuccinat, Polyvinylacetatphthalat, vorzugsweise Hydroxypropylmethylcellulosephthalat.

3. Verfahren gemäß Ansprüchen 1 und 2, bei dem das in Schritt a) verwendete Lösungsmittel aus der Gruppe der Ketone, Alkohole, Ether oder einer Mischung davon oder einer Mischung davon mit Wasser ausgewählt ist

4. Verfahren gemäß Anspruch 3, bei dem das in Schritt a) verwendete Lösungsmittel eine Mischung von Aceton und Wasser, oder eine Mischung von Aceton und Methanol, ist.

5. Verfahren gemäß mindestens einem der Ansprüche 1 bis 4, bei dem das Fällungsmedium in Schritt b) aus polaren protischen oder unpolaren aprotischen Lösungsmitteln ausgewählt ist.

6. Verfahren gemäß mindestens einem der Ansprüche 1 bis 5, bei dem das Fällungsmedium in Schritt b) aus Wasser und Heptan, vorzugsweise Wasser, ausgewählt ist.

7. Verfahren gemäß mindestens einem der Ansprüche 1 bis 6, bei dem das Fällungsmedium in Schritt b) Wasser bei einer Temperatur unter 20 °C, vorzugsweise unter 10 °C, am meisten bevorzugt unter 5 °C, ist.

8. Co-Präzipitat, erhältlich durch mindestens einen der Ansprüche 1 bis 7, **gekennzeichnet durch** eine spezifische Oberfläche von mehr als 6 m²/g, vorzugsweise mehr als 10 m²/g und am meisten bevorzugt mehr als 15 m²/g.

9. Pharmazeutische Zusammensetzung umfassend ein Tadalafil-Co-Präzipitat und mindestens ein wasserlösliches Verdünnungsmittel und/oder wasserunlösliches nichtquellbares Verdünnungsmittel, wobei die Zusammensetzung im Wesentlichen frei von wasserunlöslichen quellbaren Verdünnungsmitteln ist, so dass der Gehalt an wasserunlöslichen quellbaren Verdünnungsmitteln weniger als 5 Gew.-%, bezogen auf das Tablettengewicht, beträgt.

10. Pharmazeutische Zusammensetzung gemäß Anspruch 9, wobei der Gehalt an wasserlöslichem Verdünnungsmittel und/oder wasserunlöslichem nicht-quellbaren Verdünnungsmittel von 50 Gew.-% bis 95 Gew.-% pro Tablettengewicht, vorzugsweise 55 bis 90 Gew.-% pro Tablettengewicht, am meisten bevorzugt 60 bis 85 Gew.-% pro Tablettengewicht, variieren kann.

11. Pharmazeutische Zusammensetzung gemäß Anspruch 9 oder 10, wobei der Gehalt an wasserunlöslicher weiterer Komponente weniger als 10 Gew.-%, am meisten bevorzugt weniger als 5 Gew.-%, bezogen auf das Tablettengewicht, beträgt.

12. Pharmazeutische Zusammensetzung gemäß mindestens einem der Ansprüche 9 bis 11, wobei das wasserlösliche Verdünnungsmittel ausgewählt ist aus der Gruppe, umfassend Kohlenhydrate wie Dextrate; Polysaccharide wie Maltodextrin; Monosaccharide wie Glucose und Fructose; Oligosaccharide wie Saccharose und Lactose, wie wasserfreie Lactose, amorphe Lactose, sprühgetrocknete Lactose, granulierte Laktose und Laktosemonohydrat und Zuckeralkohole wie Mannitol, Erythritol, Sorbitol, Dulcitol, Ribitol und Xylitol.

13. Pharmazeutische Zusammensetzung gemäß mindestens einem der Ansprüche 9, 10 und 12, wobei das wasserunlösliche quellbare Verdünnungsmittel ausgewählt ist aus der Gruppe, umfassend Stärken wie Maisstärke, Kartoffelstärke, Weizenstärke und teilweise vorgelatinierte Stärken; Cellulose und ihre Derivate wie mikrokristalline Cellulose, verkieselte mikrokristalline Cellulose und pulverisierte Cellulose.

14. Pharmazeutische Zusammensetzung gemäß mindestens einem der Ansprüche 9 bis 13, wobei das wasserlösliche Verdünnungsmittel Mannitol ist und wobei die Zusammensetzung im Wesentlichen frei und vorzugsweise vollständig frei von mikrokristalliner Cellulose ist.

## Revendications

1. Procédé de préparation de coprécipités de tadalafil avec des excipients pharmaceutiquement acceptables qui comprend les étapes suivantes :
a) dissolution du tadalafil et au moins un excipient pharmaceutiquement acceptable dans un solvant organique ou un mélange de ceux-ci ou un mélange de ceux-ci avec de l'eau à température et/ou pression élevée(s),
b) ajout de la solution obtenue à l'étape a) dans un milieu de précipitation à une température allant de 0 °C à 40 °C, de préférence de 0 °C à 20 °C, plus préférentiellement de 0 °C à 10 °C, le plus préférentiellement de 0 °C à 5 °C et à une pression allant de la pression atmosphérique normale à une pression de 0,6 MPa, de préférence à une pression atmosphérique normale à 0,4 MPa, ou à une pression située dans la plage allant de 0,11 MPa à 0,6 MPa et de préférence de 0,12 MPa à 0,6 MPa,
c) facultativement, la suspension formée à l'étape b) peut être chauffée à une température de reflux et les solvants peuvent être au moins partiellement éliminés par distillation,
d) isolement du coprécipité obtenu.

2. Procédé selon la revendication 1, dans lequel l'excipient pharmaceutiquement acceptable utilisé à l'étape a) peut être choisi parmi un phtalate d'hydroxypropylméthylcellulose (HPMCP), un succinate d'acétate d'hydroxypropyl-méthylcellulose, un phtalate de polyacétate de vinyle, de préférence un phtalate d'hydroxypropylméthylcellulose.

3. Procédé selon les revendications 1 et 2, dans lequel le solvant utilisé à l'étape a) est choisi parmi le groupe des cétones, des alcools, des éthers ou d'un mélange de ceux-ci, ou d'un mélange de ceux-ci avec de l'eau.

4. Procédé selon la revendication 3, dans lequel le solvant utilisé à l'étape a) est un mélange d'acétone avec de l'eau ou un mélange d'acétone avec du méthanol.

5. Procédé selon l'une quelconque des revendications 1 à 4, dans lequel le milieu de précipitation à l'étape b) est choisi parmi les solvants protiques polaires ou aprotiques non polaires.

6. Procédé selon l'une quelconque des revendications 1 à 5, dans lequel le milieu de précipitation à l'étape b) est choisi parmi l'eau et l'heptane, de préférence l'eau.

7. Procédé selon l'une quelconque des revendications 1 à 6, dans lequel à l'étape b), le milieu de précipitation est de l'eau à une température inférieure à 20 °C, de préférence inférieure à 10 °C, le plus préférentiellement inférieure à 5 °C.

8. Coprécipité pouvant être obtenu par l'une quelconque des revendications 1 à 7, **caractérisé par** une surface spécifique supérieure à 6 m²/g, de préférence supérieure à 10 m²/g et le plus préférentiellement supérieure à 15 m²/g.

9. Composition pharmaceutique comprenant un coprécipité de tadalafil et au moins un diluant soluble dans l'eau et/ou un diluant incapable de gonfler insoluble dans l'eau, dans laquelle la composition est sensiblement exempte de diluants capables de gonfler insolubles dans l'eau de telle sorte que la teneur en diluants capables de gonfler insolubles dans l'eau est inférieure à 5 % en poids par poids de comprimé.

10. Composition pharmaceutique selon la revendication 9, dans laquelle la teneur en diluant soluble dans l'eau et/ou en diluant incapable de gonfler insoluble dans l'eau peut varier de 50 % à 95 % en poids par poids de comprimé, de préférence de 55 % à 90 % en poids par poids de comprimé, le plus préférentiellement de 60 % à 85 % en poids par poids de comprimé.

11. Composition pharmaceutique selon les revendications 9 ou 10, dans laquelle la teneur en composant supplémentaire insoluble dans l'eau est inférieure à 10 % en poids, le plus préférentiellement inférieure à 5 % en poids par poids de comprimé.

12. Composition pharmaceutique selon les revendications 9 à 11, dans laquelle le diluant soluble dans l'eau est choisi parmi le groupe comprenant les glucides tels que les dextrates ; les polysaccharides tels que la maltodextrine ; les monosaccharides tels que le glucose et le fructose ; les oligosaccharides tels que le saccharose et le lactose, comme le lactose anhydre, le lactose amorphe, le lactose séché par pulvérisation, le lactose granulé et le lactose monohydraté, et les alcools de sucre tels que le mannitol, l'érythritol, le sorbitol, le dulcitol, le ribitol et le xylitol.

13. Composition pharmaceutique selon les revendications 9, 10 et 12, dans laquelle le diluant capable de gonfler insoluble dans l'eau est choisi parmi le groupe comprenant les amidons tels que l'amidon de maïs, l'amidon de pomme de terre, l'amidon de blé et les amidons partiellement prégélatinisés ; la cellulose et ses dérivés tels que la cellulose microcristalline, la cellulose microcristalline silicifiée et la cellulose en poudre.

14. Composition pharmaceutique selon les revendications 9 à 13, dans laquelle le diluant soluble dans l'eau est le mannitol et dans laquelle la composition est sensiblement exempte et de préférence complètement exempte de cellulose microcristalline.
